# EUROPÄISCHE PATENTSCHRIFT

(11) **EP 4 596 987 B1**
(45) Veröffentlichungstag und Bekanntmachung des Hinweises auf die Patenterteilung: **02.12.2026**
(21) Anmeldenummer: 24210294.5
(22) Anmeldetag: 31.10.2024
(51) Int. Cl.: F24F 8/50, F24F 7/02, F24F 13/02

(54) **PERSONENSCHUTZANLAGE UND BELÜFTUNGSANLAGE FÜR EINE PERSONENSCHUTZANLAGE**
PERSONS PROTECTION SYSTEM AND VENTILATION SYSTEM FOR A PERSONS PROTECTION SYSTEM
SYSTÈME DE PROTECTION DES PERSONNES ET SYSTÈME DE VENTILATION POUR UN SYSTÈME DE PROTECTION DES PERSONNES

(43) Veröffentlichungstag der Anmeldung: 06.08.2025
(73) Patentinhaber: Andair AG, 8450 Andelfingen (CH)
(72) Erfinder: RIEDO, Lukas, 8462 Rheinau (CH); RIEDO, Michael, 8462 Rheinau (CH)
(74) Vertreter: Keller Schneider Patent- und Markenanwälte AG

(56) Entgegenhaltungen:
- CN-A- 109 594 835
- US-A- 3 795 438
- US-A- 5 924 597

## Beschreibung

### Technisches Gebiet

Die Erfindung betrifft Schutzraumanlagen mit mehreren Räumen für Katastrophensituationen. Es sollen sich mehrere Personen über eine längere Zeit im Schutzraum aufhalten können. Insbesondere betrifft die Erfindung eine Belüftungsanlage für eine solche Schutzraumanlage und ein Verfahren für den Betrieb einer solchen Personenschutzanlage.

### Stand der Technik

Schutzräume für Katastrophenfälle sollen so ausgestattet sein, die Bewohner im Notfall über eine längere Zeit (z.B. mehrere Tage oder sogar Wochen) im Schutzraum leben können. Es kann sein, dass die Bewohner in dieser Zeit auch kein Tageslicht sehen.

Aus der US 2009/217930 A1 (Holley et al.) ist ein Schutzraum für Personen bekannt. Er hat mehrere abtrennbare Räume und kann als Katastrophenschutzraum dienen. Die Räume werden mit einem Belüftungssystem klimatisiert. Mit einer Lichtsteuerung können vorprogrammierte Licht-Szenarien (Lichttemperatur, Farbe) eingestellt werden. Ferner wird die Luftfeuchtigkeit mit einer programmierten Steuerung über 24h geregelt.

Ein weiterer Personenschutzraum ist aus der US 2023/052761 (Hubbard) bekannt. Er befindet sich unter dem Erdboden und hat mehrere Räume. Ein Belüftungssystem versorgt die Räume mit Luft. Ferner ist bei Bedarf eine automatisierte Lichtsteuerung vorgesehen, um das Licht automatisch ein- und auszuschalten.

Die JP 3-240.194 U (Building System Co.) zeigt eine Schutzkonstruktion, die innerhalb von Wohngebäuden platziert wird. Sie hat bei Bedarf z.B. zwei abtrennbare Räume und kann im Fall eines Erdbebens sicher bewohnt werden. Mit einem Belüftungssystem soll der Schlaf über alle fünf Sinne gefördert werden.

Die US 2016/106056 A1 (Piccioni) beschreibt eine Schutzkammer für Tiere oder Menschen, die einen Schutz gegen Natur- und Kriegsereignisse bietet. Sie ist auf dem Boden verankert, kann aber auch unter dem Boden angeordnet sein. Es sind ein Belüftungssystem und eine Lichtsteuerung vorgesehen. Die Lichtsteuerung soll den Tag-Nacht Rhythmus vorgeben zur Regelung der biologischen Uhr der Bewohner.

Aus der Schlafforschung ist bekannt, dass mit Düften Einfluss auf das Einschlafen genommen werden kann. Die US 2022/313945 A1 (Lazarovich) beschreibt eine Pavlov'sche Schlafkonditionierungzelle mit Duftstoffkonditionierung. Das Belüftungssystem hat einen Integrierten Duft-Dispenser. Ein Sensor misst die physiologischen Parameter der Person und löst den Duft-Dispenser aus, wenn die Person im hypnagogischen Zustand ist, also im Zustand kurz vor dem Einschlafen. Nach der Konditionierung kann der Benutzer den Duftzerstäuber benutzen um

Die US 5 924 597 A zeigt eine Belüftung für ein Gebäude. Anstatt dass sich jeder Bewohner im Gebäude einen Duftdispenser in seinem Raum einrichten muss, hat das ganze Gebäude in der zentralen Heiz- und Klimaanlage (HVAC) ein Dispensersystem, um zeitlich und örtlich selektiv die gewünschten Düfte zu verteilen. Dazu hat die Anlage verschiedene Ventile und Timer.

Nachteile beim Stand der Technik:
Der Aufenthalt in einem Bunker ohne Tageslicht während mehreren Tag oder sogar Wochen ist für die Bewohner grundsätzlich belastend. Es ist auch bekannt, dass sich der zirkadiane Rhythmus im Lauf der Zeit verschiebt, wenn man kein Tageslicht hat und keine Zeitvorgaben nutzt. Mit einer zirkadianen Lichtsteuerung kann man der Tag/Nach-Rhythmus der Bewohner stabilisieren. Allerdings bleibt bei den Bewohnern eines Schutzraums ein innerer Stress bestehen, der den gesunden Schlafrhythmus stört.

### Darstellung der Erfindung

Aufgabe der Erfindung ist es, eine dem eingangs genannten technischen Gebiet zugehörende Personenschutzanlage zu schaffen, welche auch über eine längere Zeit gut bewohnbar ist.

Die Lösung der Aufgabe ist durch die Merkmale des Anspruchs 1 definiert. Gemäss der Erfindung hat die Personenschutzanlage mindestens zwei abtrennbare, separat belüftbare Räume. Die Belüftungsanlage weist folgende Komponenten auf:
a) Einen Ventilator mit einem Lufteingang und einem Luftausgang;
b) Mindestens ein Zuluftverteilsystem, das mit dem Luftausgang des Ventilators verbunden ist, um Luft in die belüftbaren Räume einzubringen;
c) Mindestens zwei Duftstoffbehälter mit unterschiedlichen Duftstoffen;
d) Mindestens einen steuerbaren Dispenser, um die mindestens zwei Duftstoffe aus den Duftstoffbehältern in das Zuluftverteilsystem einzubringen;
e) Mindestens ein zeitprogrammierbares Steuergerät zum Steuern einer Abgabe der mindestens zwei Duftstoffe in einem 24h Rhythmus,
f) wobei das Steuergerät derart zeitprogrammiert ist, dass eine tägliche Freigabe eines Duftstoffes aus mindestens einem der Dispenser zu einer definierten Startzeit zu Beginn einer definierten Schlafenszeit erfolgt und dass tägliches Stoppen der Freigabe des Duftstoffes zu einer festgelegten Stoppzeit nach der Startzeit erfolgt .

Der Ventilator wird im Normalfall mit einem Motor betrieben. Er kann aber auch so ausgestaltet sein, dass er im Notfall manuell betrieben werden kann.

Das Zuluftverteilsystem ist direkt oder indirekt (z.B: über ein Gasfilter) mit dem Luftausgang des Ventilators verbunden. Es weist mindestens eine, meistens aber zwei oder mehr Rohrleitungen bzw. Luftkanäle auf, um die Luft vom Ventilator in die mindestens zwei belüftbaren Räume zu transportieren. Das Zuluftverteilsystem umfasst alle Luftleitungen und allenfalls vorhandenen 1:n-Verzweigungen, die vom Luftausgang des Ventilators zu den Räumen führen.

Die Belüftungsanlage hat mindestens zwei Duftstoffbehälter mit unterschiedlichen Duftstoffen. Die Duftstoffbehälter sind an mindestens einem steuerbaren Dispenser angeschlossen. Der mindestens eine Dispenser bringt die mindestens zwei Duftstoffe aus den Duftstoffbehältern in das Zuluftverteilsystem ein.

Das zeitprogrammierbare Steuergerät steuert die Abgabe der mindestens zwei Duftstoffe in einem 24h Rhythmus. Das heisst, das zeitliche Schema der Duftstoffabgabe wiederholt sich jeden Tag. Das schliesst nicht aus, dass nicht alle Tage genau das gleiche Duftstoffprogramm abläuft. Im Rahmen der Erfindung ist es auch möglich, dass z.B. die Arbeitstage ein anderes Programm haben als die Wochenenden oder Feiertage.

### Vorteile

Mit der erfindungsgemässen Belüftungsanlage reduziert sich die unterschwellige Körperbelastung der Bewohner in der Personenschutzanlage. Durch geeignete Duftstoffe kann der Bewohner z.B. auf die Schlafzeit vorbereitet werden. Ebenso kann während des Tages über den Geruchssinn eine bestimmte Aktivität signalisiert werden, beispielsweise eine Zeit konzentrierten Arbeitens oder eine Pause. Dabei kann die Duftkonditionierung genutzt werden, wie sie aus wissenschaftlichen Untersuchungen bekannt ist (vgl. z..B. US 2022/313945 A1, Lazarovich).

Die erfindungsgemässe Anlage hat weiter den Vorteil, dass auf unterschiedliche Verhaltensschemata unterschiedlicher Bewohnergruppen eingegangen werden kann. So kann es in einer Notfallsituation wichtig sein, dass immer gewisse Bewohner aktiv sind (Arbeiten ausführen, System überwachen, Personen betreuen etc.). Das heisst, dass nicht alle zur selben Zeit schlafen gehen oder aufwachen. Indem mindestens zwei verschiedene Duftstoffe z.B: in unterschiedliche Räume und/oder zu unterschiedlichen Zeiten ausgetragen werden können, wird die Vielfalt bzw. gruppenspezifische Anpassung ermöglicht.

Die erfindungsgemässe Duftsteuerung kann selbst zur Stabilisierung des Zirkadianen Rhythmus, insbesondere des Schlafrhythmus, eingesetzt werden. Sie kann mit einer zirkadianen Lichtsteuerung kombiniert werden. Insgesamt verbessert sich die Lebensqualität in einem Personenschutzraum.

### Besondere Ausführungsart Nr. 2:

Gemäss einer besonderen Ausführungsart der Erfindung sind die Duftstoffe arzneimittelfrei. Das heisst, es werden keine narkotisierenden Stoffe verwendet, um das Einschlafen zu bewirken. Damit ist sichergestellt, dass die Gesundheit der Bewohner nicht durch Nebenwirkungen von Wirkstoffen beeinträchtigt wird.

Wenn in der Personenschutzanlage ein Spital betrieben wird, kann es unter Umständen durchaus Sinn machen, narkotisierende Stoffe für die Patienten in einem Raum zu verwenden. Die Erfindung schliesst also die Verwendung von arzneimittelhaltigen Duftstoffen (Schlafmitteln) nicht aus.

Gemäss einer ersten bevorzugten Variante der besonderen Ausführungsart Nr. 2 ist mindestens einer der Duftstoffe ein Entspannungsduftstoff.

Der Entspannungsduftstoff kann beispielsweise durch eine Schlafkonditionierung gewonnen werden, wie sie aus US 2022/313945 A1 (Lazarovich) bekannt ist.

Gemäss einer zweiten bevorzugten Variantre der besonderen Ausführungsart Nr. 2 ist mindestens einer der Duftstoffe ein Aktivierungsduftstoff.

### Besondere Ausführungsart Nr. 3:

Bei einer besonderen Ausführungsart sind bei der Belüftungsanlage der Personenschutzanlage die mindestens zwei Duftstoffbehälter an dem mindestens einen steuerbaren Dispenser angeschlossen. Der steuerbare Dispenser bringt also entsprechend seiner Programmierung entweder den einen Duftstoff oder den anderen Duftstoff in das Zuluftverteilsystem. Diese Ausführungsart ermöglicht es, zwei Duftstoffe mit nur einem Dispenser zu auszutragen.

Die einfachste Variante ist für Personenschutzanlagen geeignet, in welchen alle Räume zu einer bestimmten Zeit mit nur einem Duft beaufschlagt werden. Das heisst, die verschiedenen Duftstoffe werden zu verschiedenen Zeiten durch einen einzigen Dispenser in den Räumen verteilt.

Es können durchaus auch mehrere Dispenser mit je mindestens zwei Duftstoffbehältern vorgesehen sein. Damit können auch grössere Personenschutzanlagen in vielfältiger Weise im Sinn der Erfindung belüftet werden.

### Besondere Ausführungsart Nr. 4:

Bei einer weiteren Ausführungsart hat die Belüftungsanlage der Personenschutzanlage das Zuluftverteilsystem mindestens zwei Verteilkanäle und mindestens zwei Dispenser, wobei an jedem der mindestens zwei Verteilkanäle einer der steuerbaren Dispenser angeschlossen ist. Dies hat den Vorteil, dass z.B. zwei verschiedene Räume der Personenschutzanlage zeitlich völlig unabhängig voneinander mit Duftstoffen versorgt werden können. Dabei können in beiden Dispensern die gleichen verschiedenen Duftstoffe vorgesehen sein. Es ist aber auch möglich dass die Dispenser alles unterschiedliche Duftstoffe verteilen.

Es ist auch möglich, dass das Zuluftverteilsystem auch noch Verteilkanäle hat, an die kein Dispenser angeschlossen ist. So kann z.B. ein Hygieneraum (Waschroum, Toilette) frei von Duftstoffen gehalten werden. Das heisst, an den Verteilkanal zum Hygieneraum ist kein Dispenser angeschlossen.

### Besondere Ausführungsart Nr. 5:

Eine besondere Ausführungsart zeichnet sich dadurch aus, dass das Zuluftverteilsystem mindestens eine 1:n-Verzweigung bildet, wobei ein Stamm der 1:n-Verzweigung mit dem Luftausgang des Ventilators verbunden ist und wobei n Verzweigungsarme (wobei n eine ganze Zahl grösser 1 ist) Luft für die unterschiedlichen Räumen bereitstellen. Die 1:n-Verzweigung ist also im einfachsten Fall eine 1:2-Verzweigung (z.B. eine Y-Verzweigung, die einen Stamm und zwei Arme hat).

Die Zahl n kann der Anzahl belüfteter Räume in der Personenschutzanlage entsprechen. In diesem Fall ist die 1:n-Verzweigung mit Vorteil im gleichen Raum wie der Ventilator angeordnet (also z.B. in einem Maschinenraum). Ebenfalls ist es von Vorteil, wenn bei einem solchen Zuluftverteilsystem die Dispenser an den Armen der 1:n-Verzweigung angeschlossen sind, so dass auch die Dispenser zentral (z.B. im Maschinenraum) gewartet werden können.

Gemäss einer ersten bevorzugten Variante kann am Stamm der 1:n-Verzweigung der mindestens eine Dispenser angeschlossen werden.

Gemäss einer zweiten bevorzugten Variante kann an jedem der Arme der 1:n-Verzweigung mindestens ein Dispenser angeschlossen werden. Jeder der Arme der 1:n-Verzweigung versorgt einen Teil der Räume.

Die erste und die zweite bevorzugte Variante können auch kombiniert werden.

Die 1:n-Verzweigung erlaubt es, einerseits eine hohe Flexibilität bei der Verteilung unterschiedlicher Duftstoffe in unterschiedliche Räume zu haben und andererseits alle Dispenser zentral anzuordnen. Die 1:n-Verzweitung ist z.B. im Maschinenraum zusammen mit dem Ventilator angeordnet.

### Besondere Ausführungsart Nr. 6:

Gemäss einer besonderen Ausführungsart hat das Steuergerät mindestens zwei separat programmierbare Speicher, um die zwei Duftstoffe in zwei verschiedenen Zeiträumen abzugeben. Die Speicher können in einem grossen physischen Speicher als zwei separate Speicherbereich oder Speicherplätze realisiert sein. Es kann aber auch sein, dass die mindestens zwei Speicher in zwei verschiedenen Geräten vorgesehen sind. Es ist z.B. denkbar, dass jeder Dispenser mit einer Anzahl Speicherplätzen ausgestattet ist, und jeder Dispenser separat programmiert wird.

### Besondere Ausführungsart Nr. 7:

Bei einer weiteren Ausführungsart der Erfindung hat das Steuergerät einen Speicher zum Abspeichern einer definierten Schlafenszeit.

Die Programmierung der Duftstoffe zum Signalisieren der Schlafenszeit oder der Aufwachzeit kann dann relativ zur abgespeicherten Schlafenszeit erfolgen. Man braucht dann nur die Schlafenszeit festzulegen, um die Duftsteuerung zu programmieren.

Diese Ausführungsart eignet sich gut für eine zentrale Steuerung, die alle Dispenser steuert.

### Besondere Ausführungsart Nr. 8:

Bei einer besonderen Ausführungsart ist mindestens ein Verteilkanal frei von Dispensern ist, so dass zu keinem Zeitpunkt Duftstoffe in diesen Verteilkanal und die an diesen angeschlossenen Räume gelangen.

### Besondere Ausführungsart Nr. 9:

Gemäss einer weiteren Ausführungsart führen separate Abluftkanäle von jedem Raum zu einem Abluftausgang führen. Damit ist sichergestellt, dass nicht Duftstoffe aus einem Raum indirekt in einen anderen Raum gelangen können.

Wenn mehrere Räume die gleiche Funktion für die Bewohner der Personenschutzanlage haben und somit diese Räume in gleicher Weise (d.h. mit einem gemeinsamen Zeitprogramm und mit identischen Duftstoffen beaufschlagt werden), dann kann es aber auch sinnvoll sein, dass zwei oder mehrere Räume über einen Abluftkanal verbunden sind.

### Besondere Ausführungsart Nr. 10:

Bei einer besonderen Ausführungsart ist dem Ventilator ein Gasfilter vorgeschaltet. Die von ausserhalb der Personenschutzanlage zugeführte Luft wird im Luftfilter z.B. von schädlichem Aerosolen oder giftigem Gas befreit.

### Besondere Ausführungsart Nr. 11:

Weiter kann gemäss einer Ausführungsart zum Ventilator vorgeschaltet ein Explosionsschutzventil vorgesehen sein. Ein Explosionsschutzventil hält extern auftreffende Druckwellen von den Räumen der Personenschutzanlage fern. Das Explosionsschutzventil schützt auch die Belüftungsanlage (Ventilator, Dispenser etc.) vor Schäden wegen Druckstössen.

### Besondere Ausführungsart Nr. 12:

Gemäss einer besonderen Ausführungsart ist der fensterlose Betonbunker insbesondere ein unterirdischer Betonbunker. Auf diese Weise ist der Personenschutzraum besonders für Kriegsereignisse oder schwere Katastrophen geeignet.

### Besondere Ausführungsart Nr. 13:

Gemäss einer besonderen Ausführungsart hat die Personenschutzanlage einen Eingangsbereich mit einem Raum als Schmutzschleuse.

### Besondere Ausführungsart Nr. 14:

Gemäss einer besonderen Ausführungsart hat die Personenschutzanlage einen separaten Raum als Maschinenraum, in dem im Wesentlichen alle Komponenten der Belüftungsanlage untergebracht sind. Dazu gehören der Ventilator und die Dispenser, Ebenfalls kann die zentrale Steuerung der Belüftungsanlage im Maschinenraum sein, sofern eine solche Steuerung vorgesehen ist. Sofern 1:n-Verzweigungen vorgesehen sind, werden auch diese im Maschinenraum untegebracht.

### Besondere Ausführungsart Nr. 15:

Gemäss einer besonderen Ausführungsart weist die Personenschutzanlage mindestens vier Räume auf. Diese Räume sind insbesondere ein Schlafraum, ein Arbeitsraum, eine Küche und ein Hygieneraum. Es können nach Bedarf auch mehrere Räme einer bestimmten Art (Schlafraum, Arbeitsraum etc.) vorgesehen sein.

### Belüftungsanlage:

Die Erfindung bezieht sich auch auf eine Belüftungsanlage gemäss Anspruch 16, die in einer Personenschutzanlage in Form eines fensterlosen Betonbunkers mit mindestens zwei abtrennbaren, separat belüftbaren Räumen einsetzbar ist. Die Belüftungsanlage weist folgende Komponenten auf:
a) Einen Ventilator (9) mit einem Lufteingang und einem Luftausgang;
b) Mindestens ein Zuluftverteilsystem, das mit dem Luftausgang des Ventilators verbunden ist, um Luft in die belüftbaren Räume einzubringen;
c) Mindestens zwei Duftstoffbehälter für unterschiedlichen Duftstoffen;
d) Mindestens einen steuerbaren Dispenser, um die mindestens zwei Duftstoffe aus den Duftstoffbehältern in das Zuluftverteilsystem einzubringen;
e) Mindestens ein zeitprogrammierbares Steuergerät (14) zum Steuern einer Abgabe der mindestens zwei Duftstoffe in einem 24h Rhythmus.

Die Duftstoffbehälter sind z.B. auswechselbar und können Einweg- oder Mehrweggefässe sein. Entsprechend kann es sich um Verbrauchsmaterial handeln, das nicht direkt mit der Belüftungsanlage mitgeliefert wird und daher nicht direkt mit der Belüftungsanlage mitgeliefert wird.

### Verfahren:

Die Erfindung bezieht sich auch auf ein Verfahren gemäss Anspruch 19, für den Betrieb einer Personenschutzanlage mit einer Belüftungsanlage, wobei die Personenschutzanlage mindestens zwei abtrennbare, separat belüftbare Räumen hat. Das Verfahren beinhaltet folgende Schritte:
- Tägliche Freigabe eines Duftstoffes aus mindestens einem der Dispenser zu einer definierten Startzeit zu Beginn einer definierten Schlafenszeit;
- Tägliches Stoppen der Freigabe zu einer festgelegten Stopzeit nach der Startzeit..

### Besondere Ausführungsart Nr. 16:

Gemäss einer besonderen Ausführungsart des Verfahrens beträgt der durch Startzeit und Stopzeit begrenzte Zeitraum nicht mehr als ein Drittel einer vorgegebenen Schlafenszeit. Die vorgegebene Schlafenszeit liegt z.B. im Bereich von 6 bis 8 Stunden. Somit beträgt der Zeitraum für die Duftstoffabgabe (von Startzeit zu Stopzeit) z.B. maximal 2:00 h bis 2:40h. Besonders bevorzugt beträgt der Zeitraum nicht mehr als 1 Stunde.

Das Verfahren wird vorzugsweise mit einem medikamentenfreien Duftstoff durchgeführt.

Aus der nachfolgenden Detailbeschreibung und der Gesamtheit der Patentansprüche ergeben sich weitere vorteilhafte Ausführungsformen und Merkmalskombinationen der Erfindung.

### Kurze Beschreibung der Zeichnungen

Die zur Erläuterung des Ausführungsbeispiels verwendeten Zeichnungen zeigen:
- Fig. 1: ein erstes Beispiel einer Personenschutzanlage im Aufriss;
- Fig. 2: eine schematische Darstellung eines ersten Ausführungsbeispiels für die Belüftungsanlage;
- Fig. 3: Ein schematisches Zeitdiagramm für die Steuerung der Belüftungsanlage;
- Fig. 4: eine schematische Darstellung eines ersten Ausführungsbeispiels für die Belüftungsanlage;
- Fig. 5: eine schematische Darstellung eines ersten Ausführungsbeispiels für die Belüftungsanlage;
- Fig. 6: eine schematische Darstellung eines ersten Ausführungsbeispiels für die Belüftungsanlage;
- Fig. 7: ein zweites Beispiel einer Personenschutzanlage im Grundriss.

Grundsätzlich sind in den Figuren gleiche Teile mit gleichen Bezugszeichen versehen.

### Wege zur Ausführung der Erfindung

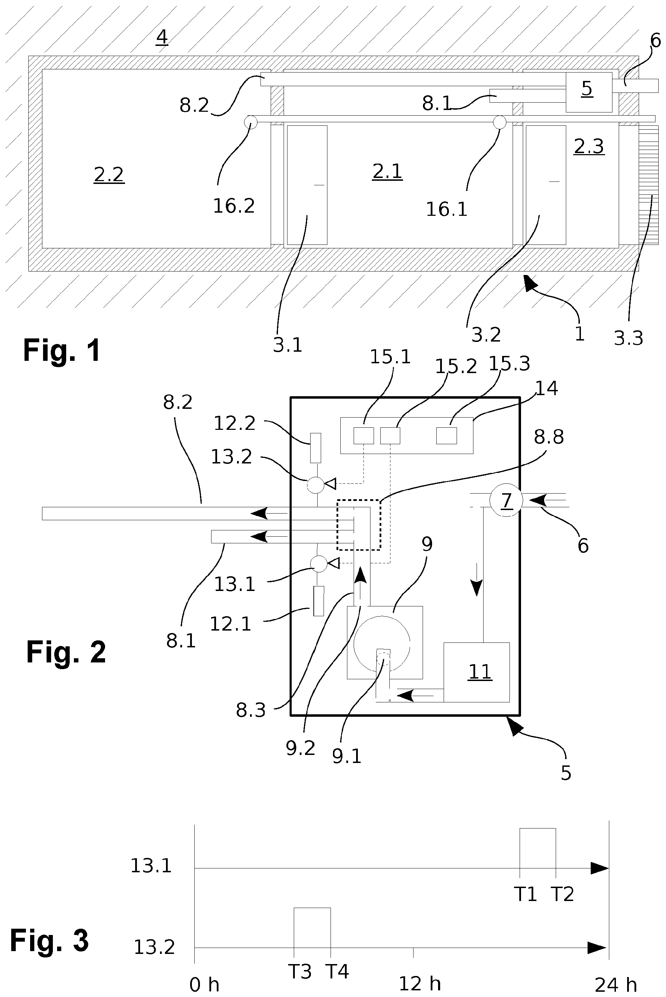

Fig. 1 zeigt schematisch eine einfache Personenschutzanlage 1. Es handelt sich hier um einen Betonbunker, der unter dem Erdreich 4 ist. Die Personenschutzanlage 1 hat beispielsweise drei Räume 2.1, 2.2, und 2.3. Der Raum 2.3 ist ein Vorraum mit einer Türe 3.3 nach aussen. Diese Türe ist z.B. eine Betontüre die luftdicht schliesst. Die drei Räume 2.1, ..., 2.3 sind über die Türen 3.1, 3.2 gegeneinander abtrennbar.

Im Vorraum 2.3 ist eine erfindungsgemässe Belüftungsanlage 5 eingebaut. Es ist einerseits über den Luftansaugkanal 6 mit der Aussenseite des Personenschutzraums 1 verbunden. Andererseits ist es über die Verteilkanäle 8.1, 8.2 mit den Räumen 2.1, 2.2 verbunden. Die über den Luftansaugkanal angesaugte Luft wird auf diese Weise in die Räume 2.1, 2.2 gebracht.

Die Abluftkanaäle 16.1, 16.2 bringen die verbrauchte Luft aus den Räumen 2.1, 2.2 nach draussen. Ebenfalls ist die erfindungsgemässe Belüftungsanlage angedeutet.

In Fig. 1 sind die besonderen Ausführungsarten Nr. 12, und Nr. 13 verwirklicht.

Fig. 2 zeigt schematisch den Aufbau einer bevorzugten Belüftungsanlage 5 Die Pfeile in den Kanälen deuten die Strömungsrichtung der Luft an.

Gemäss der besonderen Ausführungsart Nr. 11 ist zunächst ein Explosionsschutzventil 7 im Luftansaugkanal 6 vorgesehen. Danach führt der Luftansaugkanal 6 zum Gasfilter 11. Nach dem Gasfilter 11 führt der Luftansaugkanal 6 zum Lufteingang 9.1 des Ventilators 9. Der Einsatz eines Gasfilters ist

Der Luftausgang 9.2 des Ventilators 9 ist mit dem Zuluftverteilsystem verbunden. Im vorliegenden Beispiel umfasst das Zuluftverteilsystem einen Zuluftkanal 8.3 und eine 1:2-Verzweigung 8.8, wobei der Zuluftkanal 8.3 die Luft zur 1:2 Verzweigung führt. Von den Ausgängen der 1:2-Verzweigung 8.8 führen die Verteilkanäle 8.1, 8.2 die Zuluft in die Räume 2.1, 2.2. Die Verteilkanäle 8.1, 8.2 gehören im vorliegenden Beispiel ebenfalls zum Zuluftverteilsystem.

An jedem Verteilkanal 8.1, 8.2 ist ein Dispenser 13.1, 13.2 angeschlossen. Jeder Dispenser 13.1, 13.2 ist dazu ausgebildet, einen Duftstoff aus dem zugehörigen Duftstoffbehälter 12.1, 12.2 in den Verteilkanal 8.1, 8.2 einzusprühen. Die beiden Dispenser 13.1, 13.2 werden von einem Steuergerät 14 kontrolliert. Das Steuergerät 14 gibt ein Startsignal, um die Abgabe des Duftstoffs durch den Dispenser 13.1 buw. 13.2 zu starten. Nach einer programmierten Zeit gibt das Steuergerät 14 ein Stopsignal, um die Abgtabe des Duftstoffs durch den Dispenser 13.1 bzw. 13.2 zu stoppen.

Das Steuergerät 14 hat z.B. zwei Speicher 15.1, 15.2, in denen Startzeit und Stopzeit für die zugeordneten Dispenser 13.1, 13.2 gespeichert sind. Im vorliegenden Beispiel hat das Steuergerät 14 auch noch einen Speicher 15.3 für die Schlafenszeit (Beginn und Ende der Schlafenszeit).

Die Fig. 2 veranschaulicht die besonderen Ausführungsarten Nr. 3, Nr. 4, Nr. 5, Nr. 6, Nr. 7; Nr. 10 und Nr. 11. Zudem zeit Fig. 2 die zweite bevorzugte Variante der besonderen Ausführungsart Nr. 5.

Fig. 3 veranschaulicht die Steuerung bzw. das Verfahren zum Betreiben der Belüftungsanlage. Die Dispenser 13.1, 13.2 werden beide in einem 24-Stunden Rhythmus gesteuert. In Fig. 3 ist veranschaulicht, dass der Dispenser 13.1 den Duftstoff aus dem Duftstoffbehälter 12.1 im Zeitraum T1 bis T2 in den Verteilkanal 8.1 einträgt (z.B. T = 21 Uhr und T2 = 22 Uhr). T1 bezeichnet dabei die Startzeit und T2 die Stopzeit. Startzeit T1 und Stopzeit T2 sind z.B. im Speicher 15.1 des Steuergeräts 14 gespeichert.

Unabhängig vom Dispenser 13.1 hat der Dispenser 13.2 einen programmierten Zeitraum T3 bis T4, in welchem er den Duftstoff aus dem Duftstoffbehälter 12.2 in den Verteilkanal 8.2 einträgt. Startzeit T3 (z.B. T3 = 6 Uhr) und Stopzeit T4 (z.B. T4 = 7 Uhr) sind z.B. im Speicher 15.2 des Steuergeräts 14 gespeichert. Die horizontale Achse des Diagramms deckt einen 24-Stunden Zyklus von 0 Uhr bis 24 Uhr ab. Jeden Tag wird dieser Zyklus durchlaufen (d.h. die Steuerung arbeitet im 24-Stunden Rhythmus).

Fig. 3 veranschaulicht somit insbesondere die besondere Ausführungsart Nr. 6.

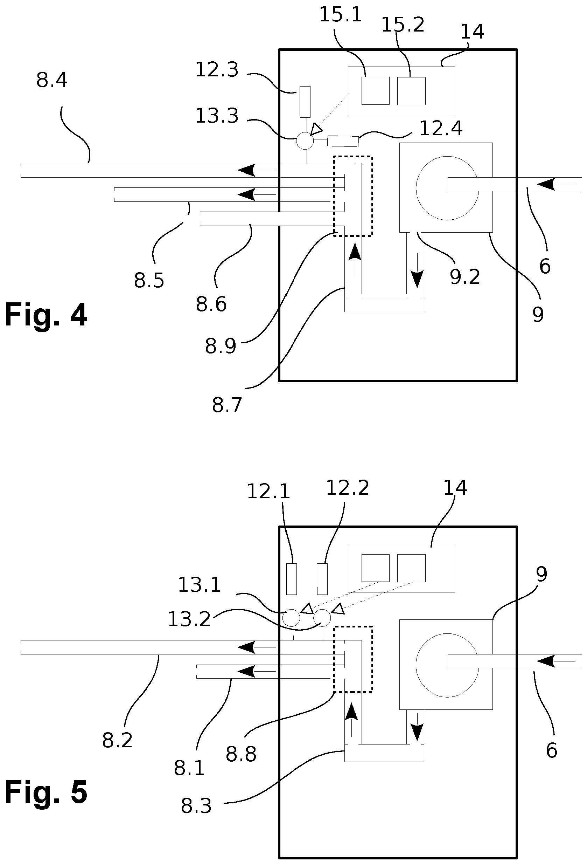

Fig. 4 zeigt schematisch einen Aufbau eines Belüftungsgeräts, das einen Dispenser 13.3 mit zwei Duftstoffbehältern 12.3, 12.4. Der Dispenser 13.3 ist am Verteilkanal 8.4 angeschlossen. Der Dispenser 13.3 ist so ausgebildet, dass er wahlweise aus dem einen Duftstoffbehälter 12.3 oder dem anderen Duftstoffbehälter 12.4 den jeweiligen Duftstoff in die Zuluft eintragen kann.

In der Fig. 4 hat das Zuluftverteilsystem einen Zuluftkanal 8.7, der an den Luftausgang 9.2 des Ventilators 9 angeschlossen ist. Der Zuluftkanal 8.7 führt die Luft zu einer 1:3-Verzweigung 8.9, welche ebenfalls ein Teil des Zuluftverteilsystems ist.. An diese 1:3-Verzweigung 8.9 sind drei Verteilkanäle 8.4, 8.5, 8.6 angeschlossen, die ebenfalls zum Zuluftverteilsystem gehören und die Luft zu drei verschiedenen Räumen (nicht dargestellt) führen. Dabei sind die drei Räume voneinander getrennt oder abtrennbar.

Gemäss der besonderen Ausführungsart Nr. 8 sind im vorliegenden Ausführungsbeispiel zwei Verteilkanäle 8.5 und 8.7 (also mindestens ein Verteilkanal) nicht mit einem Dispenser verbunden. Sie werden also nicht mit Duftstoffen beaufschlagt.

Der Verteilkanal 8.4 ist gemäss einer besonderen Ausführungsart der Erfindung zu einem Schlafraum geführt, wobei der Schlafraum als einziger Raum im programmierten 24h-Rhythmus mit Duftstoffen versehen wird. Die Zeitprogramme sind durch die beiden Speicher 15.1, 15.2 des Steuergeräts 14 gegeben.

Gemäss einer bevorzugten Ausführungsform bildet das Steuergerät14 zusammen mit dem Dispenser 13.3 eine Einheit bilden. Mit anderen Worten: Der Dispenser 13.3 kann eine integrierte Steuerung aufweisen, die es erlaubt, den Dispenser im 24h-Rhythmus zu steuern.

Fig. 5 zeigt eine Ausführungsform, bei der zwei separate Dispenser 13.1, 13.2 an einem Verteilkanal 8.1 angeschlossen sind. Der Verteilkanal 8.2 ist frei von einer Beduftung und braucht folglich auch keinen Dispenser. Die beiden Dispenser 13.1, 13.2 sind vom Steuergerät 14 kontrolliert. In den Duftstoffbehältern 12.1, 12.2 befinden sich unterschiedliche Duftstoffe. Der eine Duftstoff fördert das Einschlafen zur Schlafenszeit. Der andere Duftstoff kann das Aufwachen der Bewohner fördern.

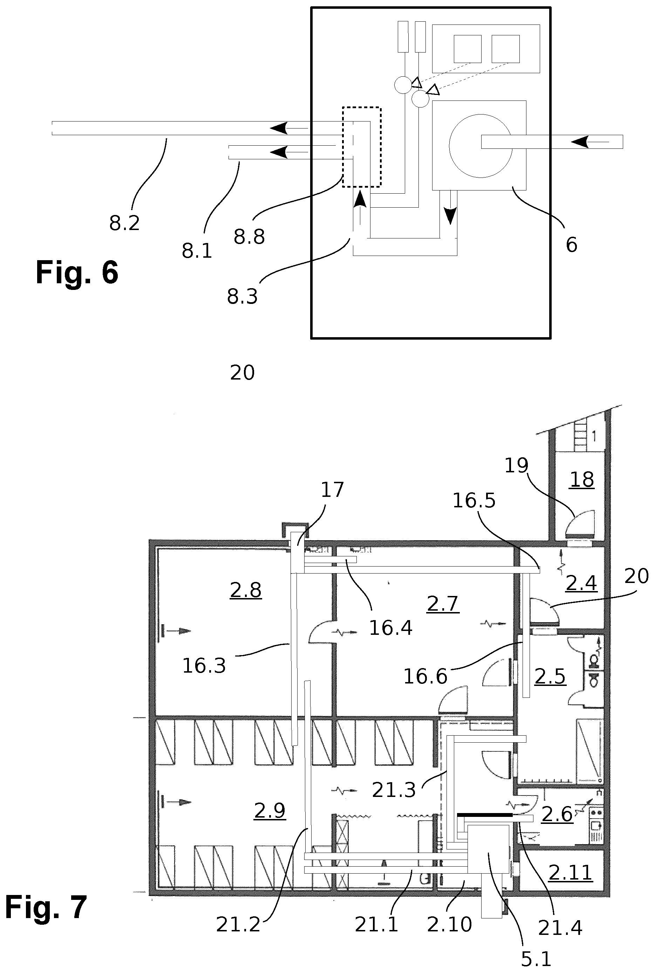

Fig. 6 zeigt eine Belüftungsanlage, bei der die Duftstoffe in den Zuluftkanal vor der 1:2-Verzweigung in die Zuluft eingebracht warden. Es sind in dieser Ausführungsform zwei Dispenser 13.1, 13.2 vorgesehen. Sie sind an den Zuluftkanal 8.3 angeschlossen. Es werden infolgedessen alle Verteilkanäle 8.1, 8.2 des Zuluftverteilsystems zu einer bestimmten Zeit den gleichen Duftstoff verteilen.

Fig. 6 veranschaulicht somit die erste bevorzugte Variante der besonderen Ausführungsart Nr. 5.

Fig. 7 zeigt eine Personenschutzanlage mit sieben abtrennbaren, separate belüftbaren Räumen. Vor der Eingangstür 19 (die vorzugsweise eine katastrophensichere Betontüre ist) befindet sich ein geschützter (insbesondere überdachter) Vorplatz 18. Es ist in der Fig. 7 eine Treppe angedeutet, die vom Bodenniveau der Umgebung zu der unter dem Boden angeordneten Personenschutzanlage führt. Die Eingangstür 19 führt zu einem Vorraum (Raum 2.4). Dieser ist durch eine Sicherheitstüre 20 von einem nachfolgenden Arbeitsraum (Raum 2.5) getrennt. Vom Raum 2.5 gelangt man über eine Türe in einen Vorraum eines Schlafsaals (Raum 2.9). Vom Raum 2.5 gelangt man über eine weitere Türe in einen ersten Mehrzweckraum (Raum 2.7). Von diesem ersten Raum 2.7 gelangt man durch Türen einerseits in einen zweiten Mehrzweckraum (Raum 2.8) und andererseits in den Vorraum des Schlafsaals (Raum 2.9). Schliesslich gibt es noch eine Küche (Raum 2.6), einen Maschinenraum (Raum 2.10) und einen (unbelüfteten) Tankraum (Raum 2.11).

Die geometrische Anordnung und die Verbindung der Räume hängt vom Zweck der Personenschutzanlage und dem verfügbaren Platz für die Personenschutzanlage ab. Für die Erfindung ist nur relevant, dass mindestens zwei abtrennbare und separate belüftete Räume 2.4, ..., 2.10 vorhanden sind.

Im Maschinenraum befinden sich der Ventilator, das dem Ventilator vorgeschaltete Luftansaugsystem (vorzugsweise mit Gasfilter), die 1:n-Verzweigung (hier eine 1:4-Verzweigung) und ein Anfangsabschnitt der Rohre des Zuluftverteilsystems.

Gemäss einer besonderen Ausführungsart ist die 1:n-Verzweigung im Belftungsgerät 5.1 untergebracht.

Die vier Arme der 1:4-Verzweigung sind mit den Zuluftverteilrohren 21.1 bis 21.4 verbunden. Ein erstes Zuluftverteilrohr 21.1 führt Frischluft in den Raum 2.9. Ein weiteres Zuluftverteilrohr 21.2 führt Friscluft in den Raum 2.8. Ein drittes und ein viertes Zuluftverteilrohr 21.3 und 21.4 führen Frischluft in die Räume 2.5 resp. 2.6.

Weiter sind in Fig. 7 vier separate Abluftkanäle vorgesehen, nämlich ein Abluftkanal 16.3 für den Raum 2.8, ein Abluftkanal 16.4 für den Raum 2.7, ein Abluftkanal 16.5 für den Raum 2.4 und ein Abluftkanal 16.6 für den Raum 2.5 vorgesehen. Entsprechend der besonderen Ausführungsart Nr. 9 ist somit für jeden separate belüftbauren Raum eine separate Abluftleitung vorgesehen, der die Abluft zu einem Abluftausgang 17 der Personenschutzanlage führt.

Im Abluftausgang 17 kann ebenfalls ein Explosionsschutzventil vorgesehen sein, wie im Zulufteingang. Zuluft und Abluft sind damit gegen Druckwellenstösse von aussen geschützt.

Zusammenfassend ist festzustellen, dass die Erfindung eine Möglichkeit zur Stabilisierung des Zirkadianen Rhythmus in Personenschutzanlagen ohne Tageslicht-Einstrahlung bietet.

### Bezugszeichenliste

- 1: Personenschutzraum [fensterlos]
- 2.1 - 2.11: Raum
- 3.1 - 3.3: Türe
- 4: Erdreich
- 5: Belüftungsanlage
- 6: Luftansaugkanal
- 7: Explosionsschutzventil
- 8.1, 8.2, 8.4, 8.5.: 8.6 Verteilkanal
- 8.3, 8.7: Zuluftkanal
- 8.8: 1:2-Verzweigung
- 8.9: 1:3-Verzweigung
- 9: Ventilator
- 9.1: Lufteingang
- 9.2: Luftausgang
- 10: ---
- 11: Gasfilter
- 12.1, 12.2: Duftstoffbehälter
- 13.1, ..,13.3: Dispenser
- 14: Steuergerät
- 15.1, 15.3: Speicher
- 16.1, ...,16.5: Abluftkanal
- 17: Abluftausgang
- 18: Vorplatz
- 19: Eingangstüre
- 20: Sicherheitstüre
- 21.1, ... 21.4: Zuluftverteilrohr

## Patentansprüche

1. Personenschutzanlage (1) in Form eines fensterlosen Betonbunkers mit mindestens zwei abtrennbaren, separat belüftbaren Räumen (2.1, ..., 2.3), und mit einer Belüftungsanlage mit folgenden Komponenten:
a) Einen Ventilator (9) mit einem Lufteingang (9.1) und einem Luftausgang (9.2);
b) Mindestens einem Zuluftverteilsystem (8.1, ..., 8.9), das mit dem Luftausgang (9.2) des Ventilators (9) verbunden ist, um Luft in die belüftbaren Räume (2.1, .., 2.10) einzubringen;
c) Mindestens zwei Duftstoffbehälter (12.1, 12.2) mit unterschiedlichen Duftstoffen;
d) Mindestens einen steuerbaren Dispenser (13.1, 13.2), um die mindestens zwei Duftstoffe aus den Duftstoffbehältern (12.1, 12.2) in das Zuluftverteilsystem (8.1, 8.2, 8.3) einzubringen;
e) Mindestens ein zeitprogrammiertes Steuergerät (14), das eine Abgabe (T1, ..., T4) der mindestens zwei Duftstoffe in einem 24-Stunden Rhythmus steuert
f) Wobei das Steuergerät (14) derart zeitprogrammiert ist, dass eine tägliche Freigabe eines Duftstoffes aus mindestens einem der Dispenser zu einer definierten Startzeit zu Beginn einer definierten Schlafenszeit erfolgt und dass tägliches Stoppen der Freigabe des Duftstoffes zu einer festgelegten Stoppzeit nach der Startzeit erfolgt.

2. Personenschutzanlage nach Anspruch 1, **dadurch gekennzeichnet, dass** die Duftstoffe arzneimittelfrei sind.

3. Personenschutzanlage nach einem der vorhergehenden Ansprüche, **dadurch gekennzeichnet, dass** die mindestens zwei Duftstoffbehälter (12.1, 12.2) an dem mindestens einen steuerbaren Dispenser (13.3) angeschlossen sind.

4. Personenschutzanlage nach einem der vorhergehenden Ansprüche, **dadurch gekennzeichnet, dass** das Zuluftverteilsystem mindestens zwei Verteilkanäle (8.1, 8.2) aufweist und dass mindestens zwei Dispenser (13.1, 13.2) vorgesehen sind, wobei an jedem der mindestens zwei Verteilkanäle (8.1, 8.2) einer der steuerbaren Dispenser (13.1, 13.2) angeschlossen ist.

5. Personenschutzanlage nach einem der vorhergehenden Ansprüche, **dadurch gekennzeichnet, dass** das Zuluftverteilsystem mindestens eine 1:n-Verzweigung hat, wobei ein Stamm der 1:n-Verzweigung mit dem Luftausgang des Ventilators (9) verbunden ist und wobei n Verzweigungsarme der 1:n-Verzweigung, wobei n eine ganze Zahl grösser 1 ist, Luft für die unterschiedlichen Räumen zur Verfügung stellen.

6. Personenschutzanlage nach einem der vorhergehenden Ansprüche, **dadurch gekennzeichnet, dass** das Steuergerät (14) zwei separat programmierbare Speicher (15.1, 15.2) aufweist, um die zwei Duftstoffe in zwei verschiedenen Zeiträumen (T1-T2, T3-T4) abzugeben.

7. Personenschutzanlage nach einem der vorhergehenden Ansprüche, **dadurch gekennzeichnet, dass** das Steuergerät (14) einen Speicher (15.3) zum Abspeichern einer definierten Schlafenszeit hat.

8. Personenschutzanlage nach einem der vorhergehenden Ansprüche, **dadurch gekennzeichnet, dass** mindestens ein Verteilkanal (8.3) frei von Dispensern ist, so dass zu keinem Zeitpunkt Duftstoffe in diesen Verteilkanal (8.3) gelangen.

9. Personenschutzanlage nach einem der vorhergehenden Ansprüche, **dadurch gekennzeichnet, dass** separate Abluftkanäle (16.1, 16.2) von jedem Raum (2.1, 2.2) zu einem Abluftausgang (17) führen.

10. Personenschutzanlage nach einem der vorhergehenden Ansprüche, **dadurch gekennzeichnet, dass** eingangsseitig des Ventilators (9) ein Gasfilter (11) vorgesehen ist.

11. Personenschutzanlage nach einem der vorhergehenden Ansprüche, **dadurch gekennzeichnet, dass** am Lufteingang (9.1) des Ventilators (9) ein Luftansaugkanal (6) angeschlossen ist, der ein Explosionsschutzventil (7) aufweist.

12. Personenschutzanlage nach dem vorhergehenden Anspruch, **dadurch gekennzeichnet, dass** der Fensterlose Betonbunker insbesondere ein unterirdischer Betonbunker ist.

13. Personenschutzanlage nach dem vorhergehenden Anspruch, **dadurch gekennzeichnet, dass** sie einen Eingangsbereich mit einem Raum als Schmutzschleuse hat.

14. Personenschutzanlage nach dem vorhergehenden Anspruch, **dadurch gekennzeichnet, dass** sie einen separaten Raum (2.10) als Maschinenraum aufweist, in dem im Wesentlichen alle Komponenten der Belüftungsanlage untergebracht sind.

15. Personenschutzanlage nach einem der vorhergehenden Ansprüche, **dadurch gekennzeichnet, dass** sie mindestens vier Räume aufweist, insbesondere einen Schlafraum, einen Arbeitsraum, eine Küche und einen Hygieneraum.

16. Belüftungsanlage (5) geeignet für eine Personenschutzanlage in Form eines fensterlosen Betonbunkers mit mindestens zwei abtrennbaren, separat belüftbaren Räumen (2.1, ..., 2.3), wobei die Belüftungsanlage folgende Komponenten aufweist:
a) Einen Ventilator (9) mit einem Lufteingang (9.1) und einem Luftausgang (9.2);
b) Mindestens ein Zuluftverteilsystem (8.1, 8.2), das mit dem Luftausgang (9.2) des Ventilators (9) verbunden (8.3) ist, um Luft in die belüftbaren Räume (2.1, .., 2.3) einzubringen;
c) Mindestens zwei Duftstoffbehälter (12.1, 12.2) für unterschiedliche Duftstoffe;
d) Mindestens einen steuerbaren Dispenser (13.1, 13.2), um die mindestens zwei Duftstoffe aus den Duftstoffbehältern (12.1, 12.2) in das Zuluftverteilsystem (8.1, 8.2, 8.3) einzubringen;
e) Mindestens ein zeitprogrammiertes Steuergerät (14), das eine Abgabe der mindestens zwei Duftstoffe in einem 24-Stunden Rhythmus steuert,
f) wobei das Steuergerät (14) derart zeitprogrammiert ist, dass eine tägliche Freigabe eines Duftstoffes aus mindestens einem der Dispenser zu einer definierten Startzeit zu Beginn einer definierten Schlafenszeit erfolgt und dass tägliches Stoppen der Freigabe des Duftstoffes zu einer festgelegten Stoppzeit nach der Startzeit erfolgt.

17. Belüftungsanlage (5) nach dem vorhergehenden Anspruch, **dadurch gekennzeichnet, dass** die mindestens zwei Duftstoffbehälter (12.1, 12.2) an dem mindestens einen steuerbaren Dispenser (13.3) angeschlossen sind.

18. Belüftungsanlage (5) nach einem der Ansprüche 17 oder 18, **dadurch gekennzeichnet, dass** das Zuluftverteilsystem mindestens zwei Verteilkanäle (8.1, 8.2) aufweist und dass mindestens zwei Dispenser 13.1, 13.2) vorgesehen sind, wobei an jedem der mindestens zwei Verteilkanäle (8.1, 8.2) einer der steuerbaren Dispenser (13.1, 13.2) angeschlossen ist.

19. Verfahren für den Betrieb einer Personenschutzanlage nach einem der Ansprüche 1 bis 15 respektive zum Betrieb einer Belüftungsanlage nach einem der Ansprüche 17 bis 19 mit folgenden Schritten:
• Tägliche Freigabe eines Duftstoffes aus mindestens einem der Dispenser zu einer definierten Startzeit zu Beginn einer definierten Schlafenszeit;
• Tägliches Stoppen der Freigabe zu einer festgelegten Stoppzeit nach der Startzeit..

20. Verfahren nach dem vorhergehenden Anspruch, **dadurch gekennzeichnet, dass** ein durch Startzeit und Stoppzeit begrenzter Zeitraum nicht mehr als ein Drittel einer vorgegebenen Schlafenszeit beträgt.

21. Verfahren nach dem vorhergehenden Anspruch, **dadurch gekennzeichnet, dass** der Duftstoff medikamentenfrei ist.

## Claims

1. Personal protection system (1) in the form of a windowless concrete bunker with at least two separable, separately ventilable rooms (2.1, ..., 2.3), and with a ventilation system comprising the following components:
a) A fan (9) with an air inlet (9.1) and an air outlet (9.2);
b) At least one supply air distribution system (8.1, ..., 8.9) connected to the air outlet (9.2) of the fan (9) for introducing air into the ventilable rooms (2.1, ..., 2.10);
c) At least two fragrance containers (12.1, 12.2) with different fragrances;
d) At least one controllable dispenser (13.1, 13.2) for introducing the at least two fragrances from the fragrance containers (12.1, 12.2) into the supply air distribution system (8.1, 8.2, 8.3);
e) At least one time-programmed control unit (14) that controls the release (T1, ..., T4) of the at least two fragrances in a 24-hour cycle
f) wherein the control unit (14) is time-programmed such that a fragrance is released daily from at least one of the dispensers at a defined start time at the beginning of a defined sleep period, and such that the release of the fragrance is stopped daily at a specified stop time following the start time.

2. Personal protection system according to claim 1, **characterized in that** the fragrances are free of pharmaceuticals.

3. Personal protection system according to one of the preceding claims, **characterized in that** the at least two fragrance containers (12.1, 12.2) are connected to the at least one controllable dispenser (13.3).

4. Personal protection system according to one of the preceding claims, **characterized in that** the supply air distribution system comprises at least two distribution channels (8.1, 8.2) and that at least two dispensers (13.1, 13.2) are provided, wherein to each of the at least two distribution channels (8.1, 8.2) one of the controllable dispensers (13.1, 13.2) is connected.

5. Personal protection system according to one of the preceding claims, **characterized in that** the supply air distribution system has at least one 1:n branch, wherein a main branch of the 1:n branch is connected to the air outlet of the fan (9) and wherein n branch arms of the 1:n branch, where n is an integer greater than 1, supply air to the various rooms.

6. Personal protection system according to one of the preceding claims, **characterized in that** the control unit (14) comprises two separately programmable memory units (15.1, 15.2) for releasing the two fragrances during two different time periods (T1-T2, T3-T4).

7. Personal protection system according to one of the preceding claims, **characterized in that** the control unit (14) has a memory (15.3) for storing a defined sleep period.

8. Personal protection system according to one of the preceding claims, **characterized in that** at least one distribution channel (8.3) is free of dispensers, such that fragrances do not enter this distribution channel (8.3) at any time.

9. Personal protection system according to one of the preceding claims, **characterized in that** separate exhaust air ducts (16.1, 16.2) lead from each room (2.1, 2.2) to an exhaust air outlet (17).

10. Personal protection system according to one of the preceding claims, **characterized in that** a gas filter (11) is provided on the inlet side of the fan (9).

11. Personal protection system according to one of the preceding claims, **characterized in that** an air intake channel (6), which comprises an explosion-proof valve (7), is connected to the air inlet (9.1) of the fan (9).

12. Personal protection system according to the preceding claim, **characterized in that** the windowless concrete bunker in particular is an underground concrete bunker.

13. Personal protection system according to the preceding claim, **characterized in that** it has an entrance area with a room serving as a dirt lock.

14. Personal protection system according to the preceding claim, **characterized in that** it comprises a separate room (2.10) serving as a machine room, in which essentially all components of the ventilation system are housed.

15. Personal protection system according to one of the preceding claims, **characterized in that** it comprises at least four rooms, in particular a sleeping room, a workroom, a kitchen, and a hygiene room.

16. Ventilation system (5) suitable for a personal protection system in the form of a windowless concrete bunker with at least two separable, separately ventilable rooms (2.1, ..., 2.3), wherein the ventilation system comprises the following components:
a) A fan (9) with an air inlet (9.1) and an air outlet (9.2);
b) At least one supply air distribution system (8.1, 8.2) connected (8.3) to the air outlet (9.2) of the fan (9) to introduce air into the ventilable rooms (2.1, .., 2.3);
c) At least two fragrance containers (12.1, 12.2) for different fragrances;
d) At least one controllable dispenser (13.1, 13.2) for introducing the at least two fragrances from the fragrance containers (12.1, 12.2) into the supply air distribution system (8.1, 8.2, 8.3);
e) At least one time-programmed control unit (14) that controls the release of the at least two fragrances in a 24-hour cycle,
f) wherein the control unit (14) is time-programmed such that a fragrance is released daily from at least one of the dispensers at a defined start time at the beginning of a defined sleep period, and such that the release of the fragrance is stopped daily at a specified stop time following the start time.

17. Ventilation system (5) according to the preceding claim, **characterized in that** the at least two fragrance containers (12.1, 12.2) are connected to the at least one controllable dispenser (13.3).

18. Ventilation system (5) according to one of claims 17 or 18, **characterized in that** the supply air distribution system comprises at least two distribution channels (8.1, 8.2) and that at least two dispensers (13.1, 13.2) are provided, wherein to each of the at least two distribution channels (8.1, 8.2) one of the controllable dispensers (13.1, 13.2) is connected.

19. Method for operating a personal protection system according to one of claims 1 to 15 respectively for operating a ventilation system according to one of claims 17 to 19, comprising the following steps:
• Daily release of a fragrance from at least one of the dispensers at a defined start time at the beginning of a defined sleep period;
• Daily stopping of the release at a specified stop time following the start time.

20. Method according to the preceding claim, **characterized in that** a time period bounded by the start time and the stop time does not exceed one-third of a predetermined sleep period.

21. Method according to the preceding claim, **characterized in that** the fragrance is drug-free.

## Revendications

1. Système de protection des personnes (1) sous la forme d'un bunker en béton sans fenêtre comprenant au moins deux espaces séparables (2.1, ..., 2.3) configurés de manière à pouvoir être ventilés séparément, ainsi qu'un système de ventilation comprenant les composants suivants :
a) un ventilateur (9) avec une entrée d'air (9.1) et une sortie d'air (9.2) ;
b) au moins un système d'alimentation en air (8.1, ..., 8.9) qui est relié à la sortie d'air (9.2) du ventilateur (9) afin d'introduire de l'air dans les espaces ventilables (2.1, ..., 2.10) ;
c) au moins deux récipients à parfum (12.1, 12.2) comprenant différents parfums ;
d) au moins un distributeur contrôlable (13.1, 13.2) afin d'introduire les au moins deux parfums contenus dans les récipients à parfum (12.1, 12.2) dans le système d'alimentation en air (8.1, 8.2, 8.3) ;
e) au moins un dispositif de commande programmé par horaire (14) qui contrôle une distribution (T1, ..., T4) des au moins deux parfums selon un cycle de 24 heures ;
f) dans lequel le dispositif de commande (14) est programmé par horaire de telle sorte qu'un parfum soit diffusé quotidiennement à partir d'au moins l'un des diffuseurs à une heure de début définie, au début d'une période de sommeil définie, et que la diffusion du parfum soit arrêtée quotidiennement à une heure de fin fixée, postérieure à l'heure de début.

2. Installation de protection des personnes selon la revendication 1, **caractérisée en ce que** les parfums sont exempts de médicaments.

3. Installation de protection des personnes selon l'une des revendications précédentes, **caractérisée en ce que** les au moins deux récipients à parfum (12.1, 12.2) sont raccordés à l'au moins un distributeur contrôlable (13.3).

4. Installation de protection des personnes selon l'une des revendications précédentes, **caractérisée en ce que** le système d'alimentation en air comprend au moins deux canaux de distribution (8.1, 8.2) et **en ce qu'**au moins deux distributeurs (13.1, 13.2) sont prévus, dans lequel l'un des distributeurs contrôlables (13.1, 13.2) est raccordé à chacun des au moins deux canaux de distribution (8.1, 8.2).

5. Installation de protection des personnes selon l'une des revendications précédentes, **caractérisée en ce que** le système d'alimentation en air comprend au moins une dérivation 1:n, dans laquelle un conduit principal de la dérivation 1:n est relié à la sortie d'air du ventilateur (9) et dans laquelle n conduits secondaires de la dérivation 1:n, n étant un nombre entier supérieur à 1, fournissent de l'air aux les différents espaces.

6. Installation de protection des personnes selon l'une des revendications précédentes, **caractérisée en ce que** le dispositif de commande (14) comprend deux mémoires programmables séparément (15.1, 15.2) pour distribuer les deux parfums au cours de deux périodes différentes (T1-T2, T3-T4).

7. Installation de protection des personnes selon l'une des revendications précédentes, **caractérisée en ce que** le dispositif de commande (14) comprend une mémoire (15.3) pour enregistrer une période de sommeil définie.

8. Installation de protection des personnes selon l'une des revendications précédentes, **caractérisée en ce qu'**au moins un canal de distribution (8.3) est exempt de distributeurs, de sorte qu'à aucun moment des parfums ne parviennent dans ce canal de distribution (8.3).

9. Installation de protection des personnes selon l'une des revendications précédentes, **caractérisée en ce que** des canaux d'évacuation d'air séparés (16.1, 16.2) mènent de chaque espace (2.1, 2.2) à une sortie d'évacuation d'air (17).

10. Installation de protection des personnes selon l'une des revendications précédentes, **caractérisée en ce qu'**un filtre à gaz (11) est prévu en amont du ventilateur (9).

11. Installation de protection des personnes selon l'une des revendications précédentes, **caractérisée en ce qu'**un canal d'aspiration d'air (6) est raccordé à l'entrée d'air (9.1) du ventilateur (9), lequel comprend une soupape de protection contre les explosions (7).

12. Installation de protection des personnes selon la revendication précédente, **caractérisée en ce que** le bunker en béton sans fenêtre est en particulier un bunker en béton souterrain.

13. Installation de protection des personnes selon la revendication précédente, **caractérisée en ce qu'**elle comprend une zone d'entrée avec un espace servant de sas anti-saleté.

14. Installation de protection des personnes selon la revendication précédente, **caractérisée en ce qu'**elle comprend un espace séparé (2.10) servant de local technique, dans lequel sont logés essentiellement tous les composants du système de ventilation.

15. Installation de protection des personnes selon l'une des revendications précédentes, **caractérisée en ce qu'**elle comprend au moins quatre espaces, en particulier un espace de couchage, un espace de travail, une cuisine et un espace d'hygiène.

16. Système de ventilation (5) approprié pour une installation de protection des personnes sous la forme d'un bunker en béton sans fenêtre comprenant au moins deux espaces séparables (2.1, ..., 2.3) configurés de manière à pouvoir être ventilés séparément, le système de ventilation comprenant les composants suivants :
a) un ventilateur (9) avec une entrée d'air (9.1) et une sortie d'air (9.2) ;
b) au moins un système d'alimentation en air (8.1, 8.2) qui est relié (8.3) à la sortie d'air (9.2) du ventilateur (9) afin d'introduire de l'air dans les espaces ventilables (2.1, ..., 2.3) ;
c) au moins deux récipients à parfum (12.1, 12.2) pour différents parfums ;
d) au moins un distributeur contrôlable (13.1, 13.2) afin d'introduire les au moins deux parfums contenus dans les récipients à parfum (12.1, 12.2) dans le système d'alimentation en air (8.1, 8.2, 8.3) ;
e) au moins un dispositif de commande programmé par horaire (14) qui contrôle une distribution des au moins deux parfums selon un cycle de 24 heures,
f) dans lequel le dispositif de commande (14) est programmé par horaire de telle sorte qu'un parfum soit diffusé quotidiennement à partir d'au moins l'un des diffuseurs à une heure de début définie, au début d'une période de sommeil définie, et que la diffusion du parfum soit arrêtée quotidiennement à une heure de fin fixée, postérieure à l'heure de début.

17. Système de ventilation (5) selon la revendication précédente, **caractérisé en ce que** les au moins deux récipients à parfum (12.1, 12.2) sont raccordés à l'au moins un distributeur contrôlable (13.3).

18. Système de ventilation (5) selon l'une des revendications 17 ou 18, **caractérisé en ce que** le système d'alimentation en air comprend au moins deux canaux de distribution (8.1, 8.2) et **en ce qu'**au moins deux distributeurs (13.1, 13.2) sont prévus, dans lequel l'un des distributeurs contrôlables (13.1, 13.2) est raccordé à chacun des au moins deux canaux de distribution (8.1, 8.2).

19. Procédé d'exploitation d'un système de protection des personnes selon l'une des revendications 1 à 15, respectivement d'un système de ventilation selon l'une des revendications 17 à 19, comprenant les étapes suivantes :
• diffusion quotidienne d'un parfum à partir d'au moins l'un des diffuseurs à une heure de début définie, au début d'une période de sommeil définie ;
• arrêt quotidien de la diffusion à une heure de fin fixée, postérieure à l'heure de début.

20. Procédé selon la revendication précédente, **caractérisé en ce qu'**une période délimitée par l'heure de début et l'heure de fin n'est pas supérieure à un tiers d'une période de sommeil prédéfinie.

21. Procédé selon la revendication précédente, **caractérisé en ce que** le parfum est exempt de médicaments.
